(19) Europäisches Patentamt
European Patent Office
Office européen des brevets

(11) Publication number: **0 027 904 A1**

# (12) EUROPEAN PATENT APPLICATION

(21) Application number: **80105817.3**

(22) Date of filing: **25.09.80**

(51) Int. Cl.³: **C 07 D 471/04,** A 61 K 31/47 // (C07D471/04, 221/00)

(30) Priority: **05.10.79 CH 9014/79**

(43) Date of publication of application: **06.05.81 Bulletin 81/18**

(84) Designated Contracting States: **AT BE CH DE FR GB IT LI LU NL SE**

(71) Applicant: **SANDOZ AG, Lichtstrasse 35, CH-4002 Basel (CH)**

(72) Inventor: **Hagenbach, Alexander, Dr., Hangelimattweg 111, CH-4148 Pfeffingen (CH)**

(54) **Phenanthroline-9-carboxylic acid derivatives, process for their production and pharmaceutical compositions containing them.**

(57) Compounds of formula I

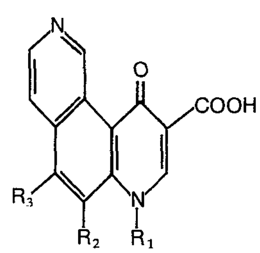

I

wherein

$R_1$ represents lower alkyl, lower alkenyl or lower hydroxyalkyl,

$R_2$ and $R_3$ represent independently of each other hydrogen, halogen, lower alkyl or lower alkoxy or salts thereof.

The compounds are useful as pharmaceuticals in particular as anti-microbial agents.

EP 0 027 904 A1

TITLE MODIFIED
see front page
0027904

Case 900-9256

# PHENANTHROLINE-9-CARBOXYLIC ACID DERIVATIVES, PROCESSES FOR THEIR PRODUCTION, PHARMACEUTICAL COMPOSITIONS CONTAINING THEM AND THEIR USE AS PHARMACEUTICALS

The invention relates to 2,7-phenanthroline-9-carboxylic acid derivatives, processes for their production, pharmaceutical compositions containing them and their use as pharmaceuticals.

GB Patent Specification No. 1,502,405 and Japanese Patent Publication No. 72/42837 broadly disclose a wide range of quinoline carboxylic acid derivatives but do not suggest compounds of the highly specific tricyclic 2,7-phenanthroline structure of the compounds of the present invention which have been found to possess particularly beneficial pharmacological properties.

The invention thus provides compounds of formula I

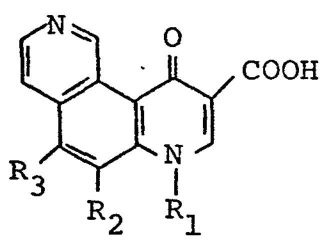

I

wherein $R_1$ represents lower alkyl, lower alkenyl or lower hydroxyalkyl,

$R_2$ and $R_3$ represent independently of each other hydrogen, halogen, lower alkyl or lower alkoxy or salts thereof.

Any lower alkyl or lower alkoxy radical has preferably 1 to 4 carbon atoms, especially 1 or 2 carbon atoms. Lower alkenyl groups contain preferably 2 to 4, in

particular 2 carbon atoms.

Halogen stands for fluorine, chlorine or bromine, preferably chlorine or bromine.

The present invention also provides a process for the production of a compound of formula I which comprises hydrolysing a compound of formula II

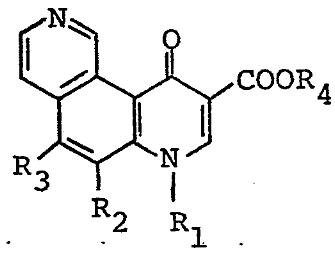

II

in which $R_1$, $R_2$ and $R_3$ are as defined above and $R_4$ represents lower alkyl.

The compounds of formula II may be produced by a process which comprises

a) reacting a compound of formula IIIa or IIIb

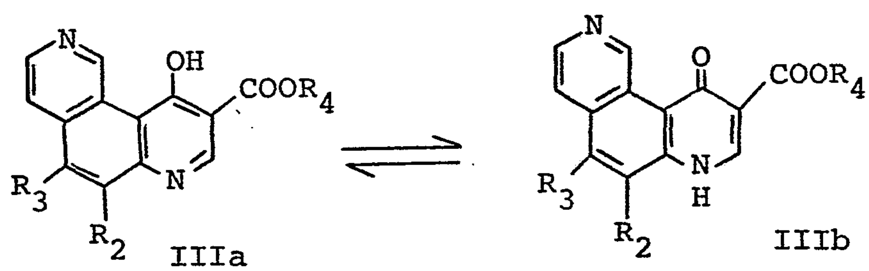

IIIa IIIb

with a compound of formula VI

$R_1X$ VI

or b) cyclising a compound of formula IVa

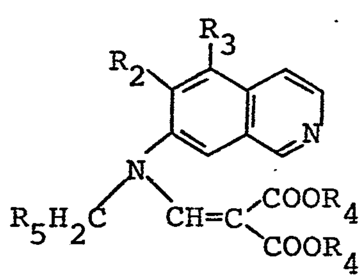

IVa

to produce a compound of formula IIa

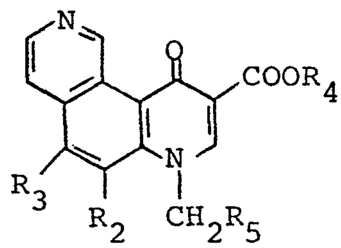

IIa

whereby in the formulae IIa, IIIa, IIIb, IVa and VI $R_1$, $R_2$, $R_3$ and $R_4$ are as defined above, $R_5$ has the same meaning as $R_1$ but contains 1 less carbon atom and X stands for an, as anion, easily removable group. Examples of such groups X are halogen and a sulphonate radical.

The hydrolysis of the compounds of formula II can be carried out analogously to known methods for the hydrolysis of esters e.g. by heating a compound of formula II with an alkaline- or alkaline earth-metalhydroxide in a suitably inert solvent such as water. The end products can be isolated and optionally purified analogously to known methods.

The cyclisation of the compounds of formula IVa can be carried out in an inert solvent e.g. in diphenylether preferably at raised temperature e.g. above 250°C.

The process a) involving compounds of formula IIIb (IIIa) may be affected in a manner conventional for the "alkylation" of secondary amines (the term "alkylation" being used here to denote the introduction of $R_1$ groups in general).

The compounds of the formula I can be converted in conventional manner into acid addition or base salts and vice versa. An example of a suitable acid is hydrochloric acid and suitable bases include alkaline or alkaline earthmetalhydroxides or ammonium hydroxide.

The starting materials other than those of formula II are either known or can be prepared analogously to known methods e.g. according to the following scheme ($R_1$ to $R_5$ being as defined above).

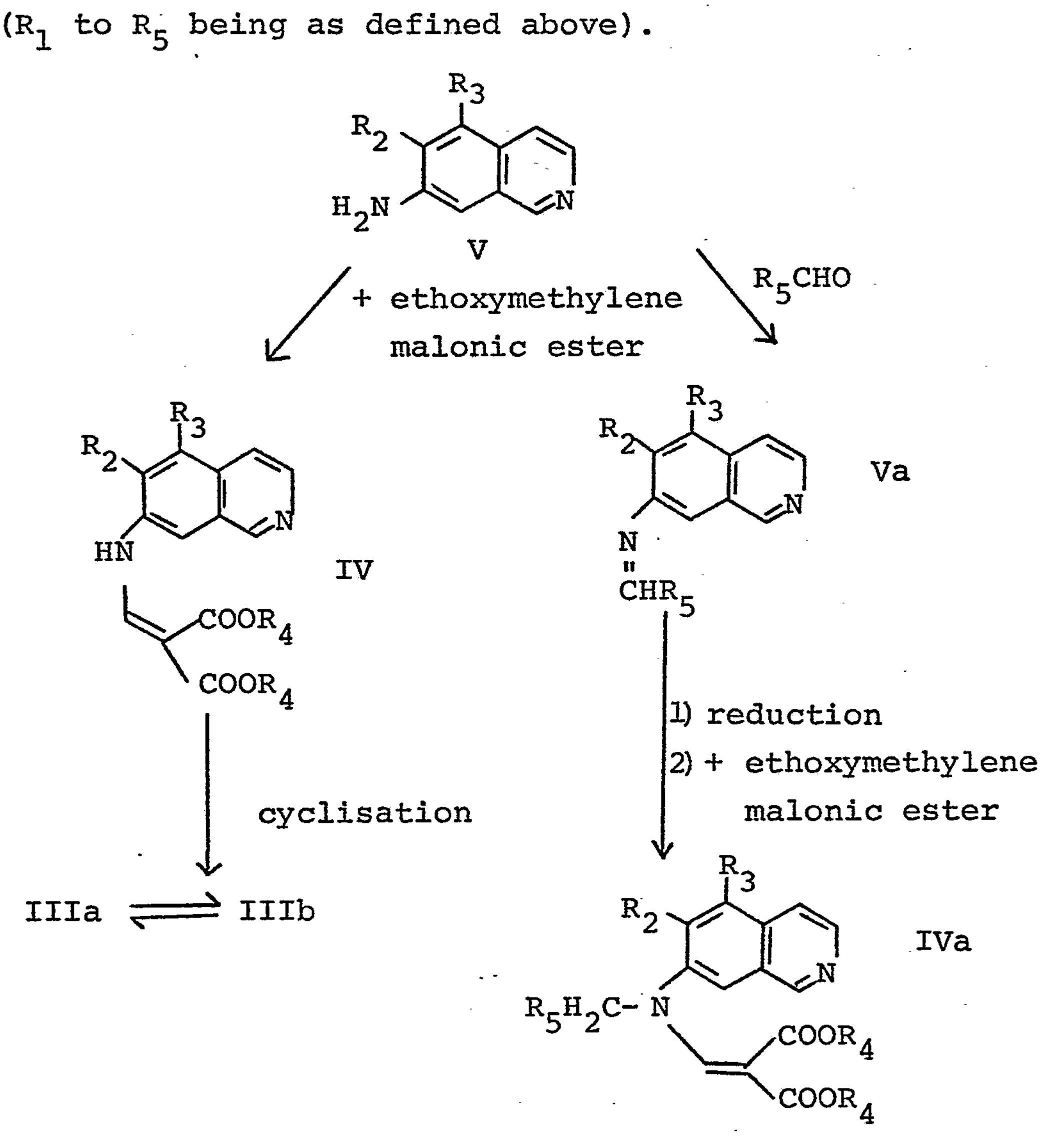

These processes are carried out in conventional manner, the cyclisation of the compounds of formula IV being analogous to that of compounds of formula IVa.

It will be appreciated that the compounds IIIa and IIIb are equivalent tautomeric forms the balance of which will depend on the nature of the substituents, and the reaction conditions employed. The compounds of formula II are new and also belong to the invention.

The compounds of formula I are useful as pharmaceuticals in particular anti-microbial agents, as indicated by their inhibiting effect against various bacteria, e.g. Staphylococcus aureus, Streptococcus pyogenes, Streptococcus faecalis, Escheria coli, Proteus vulgaris, Proteus mirabilis, Proteus morganii, Shigella spp., Enterobacter spp., Haemophilus spp., Klebsiella pneumoniae, Serrata marcescen, Salmonella spp. and Neisseria gonorrhoae, in vitro in the series dilution test at concentrations of, for example 0.01 to 50 μg/ml, and in vivo in the mouse at dosages of, for example, about 2 to 27 mg/kg of animal body weight. The compounds are therefore useful as antibacterially active antibiotics for example in treating infections of the urinary tract.

For this use, an indicated suitable daily dosage is from about 1 to 3 g, suitably administered in divided doses of from 250 to 1500 mg, two to four times daily, or in retard form.

The compounds may be employed in free form or in the form of pharmaceutically acceptable salts, which salt forms have the same order of activity as the free compounds. Suitable salt forms include alkali and alkaline earthmetal and ammonium salt forms and hydrochloride salt forms.

The compounds may be admixed with conventional pharmaceutically acceptable diluents and carriers, and administered in such forms as tablets or capsules.

Such compositions also form part of the invention. The invention therefore also concerns a method of treating diseases or infections caused by bacteria using a compound of formula I and also compounds of formula I for use as pharmaceuticals e.g. as antibiotics and for use in the treatment of the human or animal body by therapy.

The following example illustrates the invention whereby all temperatures are in degrees centigrade.

Example : 7-Ethyl-7,10-dihydro-10-oxo-2,7-phenanthroline-9-carboxylic acid

A solution of 2,5 g 7-ethyl-7,10-dihydro-10-oxo-2,7-phenanthroline-9-carboxylic acid ethyl ester in 30 ml 10% potassium hydroxide is refluxed for $2^1/2$ hours with stirring. The reaction mixture is adjusted to pH 6 with 2 N hydrochloric acid and stirred for 30 minutes at room temperature. The resulting crystals are filtered under suction and washed consecutively with ice water, acetone and ether and then dried. Colourless crystals of m.p. 286-288° are obtained. The structure is confirmed by analysis and IR, NMR, and mass spectroscopy. The compound of formula II required as starting material can be obtained as follows:

7-Ethyl-7,10-dihydro-10-oxo-2,7-phenanthroline-9-carboxylic acid ethyl ester

a) Isoquinol-7-yl-aminomethylene malonic acid diethyl ester
A mixture of 50.4 g 7-aminoisoquinoline and 79.5 g ethoxymethylene malonic acid diethyl ester is stirred under argon for 3 hours at 130°. The cooling reaction mixture is diluted with ethanol and cooled further to room temperature. The resulting crystals are filtered under suction and have a m.p. of 128-129°. Further product can be obtained from the mother liquor by recrystallization from ethanol/petrolether.

b) <u>10-Hydroxy-2,7-phenanthroline-9-carboxylic acid ethyl ester</u>

A mixture of 6,3 g isoquinol-7-yl-aminomethylene malonic acid diethyl ester and 320 ml diphenylether are placed in a 270° hot metal or oil bath. After commencement of the reaction (liberation of ethanol) the reaction mixture is allowed to stand for a further 20 minutes at 270° (bath temperature) with occasional stirring.

The mixture is cooled under argon, mixed with petrol ether, the resulting suspension filtered under suction and the residue slurried in dichloromethane. This mixture is again filtered under suction and washed with ample dichloromethane. After recrystallization from boiling dimethylformamide and careful drying in vacuo the product (light grey crystals) melts at 271-273°.

c) <u>7-Ethyl-7,10-dihydro-10-oxo-2,7-phenanthroline-9-carboxylic acid ethyl ester</u>

2.9 g Sodium hydride suspension (50 % in oil) are washed three times with petrol ether and added to 300 ml dimethylformamide. 14.8 g 10-hydroxy-2,7-phenanthroline-9-carboxylic acid ethyl ester are added at room temperature over 5 minutes with stirring and under argon. The mixture is then warmed to 80° and stirred for 30 minutes at this temperature. The reaction mixture is

cooled to room temperature and a solution of 8.58 g ethyl iodide in 100 ml dimethylformamide added dropwise over 15 minutes. Stirring is then continued for 18 hours at room temperature and 2 hours at 80°. The reaction mixture is evaporated to dryness, the residue taken up in ice water and extracted 4 times with dichloromethane. The combined dichloromethane extracts are washed with a saturated sodium chloride solution, dried over sodium sulfate, filtered and evaporated to dryness leaving a red oil which after crystallization from ethanol/ether produces crystals which melt at 184-185°. Further recrystallization from ethanol/ether produces beige crystals m.p. 189-190°.

d) <u>7-Ethyl-7,10-dihydro-10-oxo-2,7-phenanthroline-9-carboxylic acid ethyl ester</u>

7.4 g 10-hydroxy-2,7-phenanthroline-9-carboxylic acid ethyl ester and 3.8 g pulverized anhydrous potassium carbonate are suspended in 150 ml dimethylformamide stirred for 1 hour at room temperature and then warmed to 60-70°. A solution of 5.1 g ethyl bromide in 30 ml dimethylformamide is added dropwise at this temperature over 4 hours to the reaction mixture with stirring. The solvent is then removed in a rotatory evaporator (70° bath temperature), the residue taken up in ice water and extracted 5 times with ether. The combined ether extracts

contain a small quantity of O-alkylation product and are thrown away. The water phase is saturated with sodium chloride and extracted 5 times with dichloromethane. After evaporation of the solvent there remains 3.2 g of the desired ester which is purified by dissolving in dichloromethane and precipitating with diisopropyl ether. The product is the same as that of Example c).

We Claim :

1. Compounds of formula I

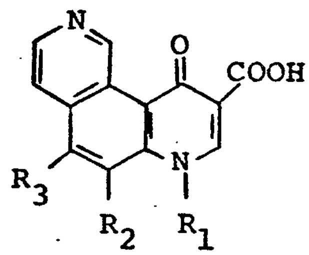

I

wherein $R_1$ represents lower alkyl, lower alkenyl or lower hydroxyalkyl,

$R_2$ and $R_3$ represent independently of each other hydrogen, halogen, lower alkyl or lower alkoxy or salts thereof.

2. 7-Ethyl-7,10-dihydro-10-oxo-2,7-phenanthroline-9-carboxylic or a salt thereof.

3. A pharmaceutical composition comprising an effective amount of a compound according to Claim 1 or 2 or a pharmaceutically acceptable salt thereof.

4. A compound according to Claim 1 or 2 for use as a pharmaceutical.

5. A compound according to Claim 1 or 2 for use as an antibiotic.

6. A compound according to Claim 1 or 2 for use in the treatment of the human or animal body for therapy.

7. A process for the production of a compound according to Claim 1 or 2 which comprises

hydrolysing a compound of formula II

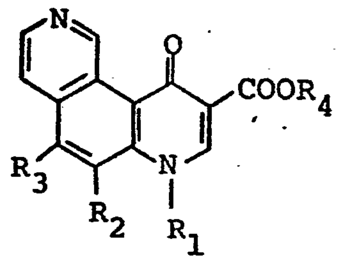

II

in which $R_1$, $R_2$ and $R_3$ are as defined above and $R_4$ represents lower alkyl.

8. All new aspects of the invention as hereinbefore described.

European Patent Office

## PARTIAL EUROPEAN SEARCH REPORT

which under Rule 45 of the European Patent Convention shall be considered, for the purposes of subsequent proceedings, as the European search report

0027904

Application number
EP 80105817.3

| DOCUMENTS CONSIDERED TO BE RELEVANT | | | CLASSIFICATION OF THE APPLICATION (Int. Cl.³) |
|---|---|---|---|
| Category | Citation of document with indication, where appropriate, of relevant passages | Relevant to claim | |
| | Patent Abstracts of Japan, unexamined applications, Section C, Vol. 3, Number 59(C-46), May 19, 1979<br>The Patent Office Japanese Government, page 30 C 46<br>+ Kokai-No. 54-32497 (TAISHO) +<br>-- | 1,7 | C 07 D 471/04<br>A 61 K 31/47//<br>(C 07 D 471/04<br>C 07 D 221/00) |
| | US - A - 3 324 135 (GEORGE Y. LESHER)<br>+ Column 1, lines 13-36; column 2, lines 10-42; column 3, lines 4-15, 23-26 +<br>---- | 1,3,7 | TECHNICAL FIELDS SEARCHED (Int. Cl.³)<br>C 07 D 471/00<br>A 61 K 31/00<br>C 07 D 221/00 |

### INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-7

Claims searched incompletely: -

Claims not searched: 8

Reason for the limitation of the search:

There is no concrete disclosure evident from claim 8

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

| Place of search | Date of completion of the search | Examiner |
|---|---|---|
| VIENNA | 11-12-1980 | ONDER |